(19) Europäisches Patentamt
European Patent Office
Office européen des brevets

(11) Veröffentlichungsnummer : **0 170 906**
**B1**

(12) # EUROPÄISCHE PATENTSCHRIFT

(45) Veröffentlichungstag der Patentschrift : **07.03.90**

(51) Int. Cl.$^5$ : **A 01 N 25/32**

(21) Anmeldenummer : **85108478.0**

(22) Anmeldetag : **09.07.85**

(54) **Pflanzenschützende Mittel auf Basis von Oximestern.**

(30) Priorität : **20.07.84 DE 3426719**

(43) Veröffentlichungstag der Anmeldung : **12.02.86 Patentblatt 86/07**

(45) Bekanntmachung des Hinweises auf die Patenterteilung : **07.03.90 Patentblatt 90/10**

(84) Benannte Vertragsstaaten : **AT BE CH DE FR GB IT LI NL**

(56) Entgegenhaltungen :
**EP-A- 0 031 938**
**EP-A- 0 094 348**
**DE-A- 2 303 336**
**DE-A- 2 808 317**
**US-A- 3 914 300**
**US-A- 3 925 473**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Kehne, Heinz, Dr.**
**Berliner Strasse 10**
**D-6238 Hofheim am Taunus (DE)**
Erfinder : **Mildenberger, Hilmar, Dr.**
**Fasanenstrasse 24**
**D-6233 Kelkheim (Taunus) (DE)**
Erfinder : **Bauer, Klaus, Dr.**
**Kolpingstrasse 7**
**D-6054 Rodgau (DE)**
Erfinder : **Bieringer, Hermann, Dr.**
**Eichenweg 26**
**D-6239 Eppstein/Taunus (DE)**

**EP 0 170 906 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Anmeldung betrifft Mittel zum Schutz von Kulturpflanzen gegen phytotoxische Nebenwirkungen von Herbiziden, dadurch gekennzeichnet, daß sie als Safener eine Verbindung der Formel I

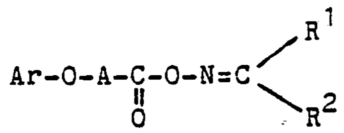

(I)

worin

Ar einen Phenyl-, Naphthyl- oder Chinolylrest, der jeweils bis zu dreifach durch Halogen und/oder ein- oder zweifach durch $CF_3$, $(C_1$-$C_4)$-Alkyl, $(C_1$-$C_4)$-Alkoxy, CN, $NO_2$, $(C_1$-$C_8$-Alkyl)-carbonyl oder Benzoyl substituiert sein kann,

A eine lineare oder verzweigte Kohlenwasserstoffkette mit bis zu vier C-Atomen,

$R^1$ H, $(C_1$-$C_6)$-Alkyl, CN, $(C_1$-$C_4$-Alkoxy)-carbonyl oder Phenyl, das durch ein bis drei Halogenatome, $(C_1$-$C_4)$-Alkyl, $(C_1$-$C_4)$-Alkoxy, $CF_3$, CN, $NO_2$, $(C_1$-$C_4$-Alkyl)-carbonyl, Benzoyl oder durch einen gegebenenfalls durch Halogen, $(C_1$-$C_4)$-Alkoxy, $(C_1$-$C_4)$-Alkyl, Nitro, $CF_3$ oder CN ein- oder mehrfach substituierten Phenyl- oder Phenoxyrest substituiert sein kann ;

$R^2$ die Bedeutung von $R^1$ außer Wasserstoff besitzt oder einen Heterocyclus mit 5 oder 6 Ringgliedern bedeutet, der ein bis drei N-, O- oder S-Atome enthalten kann, und durch Halogen oder $(C_1$-$C_4)$-Alkyl ein- bis dreifach substituiert sein kann, oder

$R^1$ und $R^2$ gemeinsam eine Alkylenkette mit 4-7 C-Atomen bedeuten, in Kombination mit Phenoxyphenoxy- oder Heteroaryloxy-phenoxy-carbonsäureester-Herbizid enthalten. Halogen bedeutet im Falle des Restes Ar insbesondere Chlor.

Bevorzugt sind diejenigen Verbindungen der Formel I, in denen

Ar Phenyl, das durch ein oder zwei Halogenatome substituiert ist, und

$-CH_2-$ oder 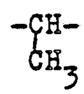

bedeuten.

Als Heterocyclen für szn Rest $R_2$ kommen insbesondere infrage Thiophen, Furan, Pyrrol, Thiazol, Pyrazol, Imidazol, Oxazol, Benzthiazol, Benzoxazol, Indol, Benzimidazol, Pyridin, Pyrimidin, Chinolin, Chinoxalin, besonders bevorzugt Thiophen, Furan und Pyridin,

Von den Verbindungen der Formel I seine beispielsweise genannt :

Aceton-O-(4-chlorphenoxyacetyl)-oxim,
Aceton-O-(3-chlorphenoxyacetyl)-oxim,
Aceton-O-(3,4-dichlorphenoxyacetyl)-oxim,
Aceton-O-(2,4-dichlorphenoxyacetyl)-oxim,
Acetophenon-O-(4-chlorphenoxyacetyl)-oxim,
Acetophenon-O-(3,4-dichlorphenoxyacetyl)-oxim,
Benzophenon-O-(4-chlorphenoxyacetyl)-oxim,
Benzophenon-O-(3,4-dichlorphenoxyacetyl)-oxim,
4-Chlorbenzophenon-O-(4-chlorphenoxyacetyl)-oxim,
4-Chlorbenzophenon-O-(3,4-dichlorphenoxyacetyl)-oxim,
2-Methyl-1-phenylpropanon-O-(4-chlorphenoxyacetyl)-oxim,
2-Methyl-1-phenylpropanon-O-(3,4-dichlorphenoxyacetyl)-oxim,
1-(4-Chlorphenyl)-2-methylpropanon-O-(4-chlorphenoxyacetyl)-oxim,
1-(4-Chlorphenyl)-2-methylpropanon-O-(3,4-dichlorphenoxyacetyl)-oxim,
Benzaldehyl-O-(4-chlorphenoxyacetyl)-oxim,
Cyclohexanon-O-(4-chlorphenoxyacetyl)-oxim,
Cyclohexanon-O-(3-chlorphenoxyacetyl)-oxim,
Cyclohexanon-O-(3,4-dichlorphenoxyacetyl)-oxim,
Cyclohexanon-O-(2,4-dichlorphenoxyacetyl)-oxim,
Cycloheptanon-O-(4-chlorphenoxyacetyl)-oxim,
Methyl-2-thienylketon-O-(4-chlorphenoxyacetyl)-oxim,
Methyl-2-thienylketon-O-(3,4-dichlorphenoxyacetyl)-oxim,
3-Pyridinaldehyd-O-(4-chlorphenoxyacetyl)-oxim,
2-(4-Chlorphenoxyacetyloximino)-propionsäureethylester,

2-(3,4-Dichlorphenoxyacetyloximino)-propionsäureethylester,
Benzophenon-O-[2-(4-chlorphenoxy)propionyl]-oxim,
Cyclohexanon-O-[2-(4-chlorphenoxy)propionyl]-oxim,
Methyl-2-thienylketon-O-[2-(4-chlorphenoxy)propionyl]-oxim.

Die Verbindungen der allgemeinen Formel I sind zum Teil bekannt oder lassen sich nach allgemein bekannten Verfahren, s. z. B. Houben Weyl, Methoden der organischen Chemie Bd. X, S. 180-187 (1968) u. Bd. XV/2, S. 137-139 (1974), herstellen. Einige der Verbindungen der Formel I wurden bereits als Herbizide beschrieben (US-PS 3 914 300 ; US-PS 3 925 473 ; DE-PS 23 03 336).

Die Verbindungen der Formel I können in syn- oder anti-Form vorliegen. Beide Isomeren werden von vorliegender Erfindung umfaßt.

Bei der Anwendung von Pflanzenbehandlungsmitteln, insbesondere Herbiziden, können unerwünschte Schäden bei den behandelten Kulturpflanzen auftreten. Besonders bei der Applikation von Herbiziden nach dem Auflaufen der Kulturpflanzen besteht daher oft das Bedürfnis, das Risiko einer möglichen Phytotoxität zu vermeiden.

Überraschenderweise wurde gefunden, daß Verbindungen der Formel I die Eigenschaften haben, phytotoxische Nebenwirkungen von Pflanzenschutzmitteln, insbesondere von Herbiziden, beim Einsatz in Nutzpflanzenkulturen zu vermindern oder ganz auszuschalten. Die Verbindungen der Formel I sind in der Lage, schädliche Nebenwirkungen der Herbizide völlig aufzuheben, ohne die Wirksamkeit dieser Herbizide gegen Schadpflanzen zu schmälern.

Solche Verbindungen, die die Eigenschaften besitzen, Kulturpflanzen gegen phytotoxische Schäden durch Herbizide zu schützen, ohne die eigentliche herbizide Wirkung dieser Mittel zu beeinträchtigen, werden « Antidote » oder « Safener » genannt.

Aus der EP-A-0 094 348 sind bereits Safener bekannt, bei denen im Vergleich zu Verbindungen der Formel (I) zumindest $R^1$ verschieden ist, d. h. fluoriertes Alkyl oder fluor-chlor-substituiertes Alkyl bedeutet. Unter der Vielzahl der dort beschriebenen Oximether werden als bevorzugt nur solche mit weit abweichender Struktur genannt.

Weiterhin sind aus der DE-A-2 808 317 Safener mit weniger verwandter Struktur bekannt, von denen keiner einen Acylrest aufweist, der denen aus Oximestern der Formel (I) ähnlich ist.

Aus der DE-A2 303 336 ist bekannt, die Kombination von Phenoxycarbonsäureoximestern, Isophoron und Herbiziden aus der Klasse der Cyanphenylcarbonate, Cyanphenoxyacetonitrile, Diurethane oder Carbamatharnstoffe zur selektiven Unkrautbekämpfung in Getreidekulturen einzusetzen.

Aus der EP-A-0 031 938 sind außerdem Aryloxyacetonitrile und -amidoxime als Safener in Kombination mit Phenoxphenoxycarbonsäureester-Herbiziden bekannt.

Das Einsatzgebiet herkömmlicher Herbizide kann durch Zugabe der Safenerverbindung der Formel I ganz erheblich vergrößert werden.

Gegenstand der vorliegenden Erfindung ist daher auch ein Verfahren zum Schutz von Kulturpflanzen gegen phytotoxische Nebenwirkungen von Phenoxyphenoxy- oder Heteroaryloxyphenoxy-carbonsäureester-Herbiziden, das dadurch gekennzeichnet ist, daß man die Pflanzen, Pflanzensamen oder Anbauflächen mit einer Verbindung der Formel I vor, nach oder gleichzeitig mit dem Herbizid behandelt.

Herbizide, deren phytotoxische Nebenwirkungen mittels Verbindungen der Formel I herabgesetzt werden können, sind Phenoxyphenoxycarbonsäureester sowie Heteroaryloxyphenoxycarbonsäureester wie Chinolyloxy-, Chinoxalyloxy-, Pyridyloxy-, Benzoxazyloxy-, Benzthiazolyloxyphenoxycarbonsäureester.

Es kommen hierbei insbesondere niedere Alkyl-, Alkenyl- und Alkinylester infrage.

Beispielsweise seien, ohne daß dadurch eine Beschränkung erfolgen soll, folgende Herbizide genannt :

Herbizide vom Typ der Phenoxyphenoxy- und Heteroaryloxyphenoxycarbonsäure-($C_1$-$C_4$)-alkyl-, ($C_2$-$C_4$)-alkenyl- und ($C_3$-$C_4$)-alkinylester wie
2-(4-(2,4-Dichlorphenoxy)-phenoxy)-propionsäuremethylester,
2-(4-(4-Brom-2-chlorphenoxy)phenoxy)-propionsäuremethylester,
2-(4-(4-Trifluormethylphenoxy)phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester,
4-(4-(4-Trifluormethylphenoxy)-phenoxy)-pent-2-entsäure-ethylester,
2-(4-(3,5-Dichlorpyridyl-2-oxy)-phenoxy)propionsäureethylester,
2-(4-3,5-Dichlorpyridyl-2-oxy)phenoxy)-propionsäurepropargylester,
2-(4-(6-Chlorbenzoxazol-2-yl-oxy)-phenoxy)-propionsäureethylester,
2-(4-(6-Chlorbenzthiazol-2-yl-oxy)-phenoxy)-propionsäureethylester,
2-(4-(3-Chlor-5-trifluormethyl-2-pyridyloxy)-phenoxy-propionsäuremethylester,
2-(4-(5-Trifluormethyl-2-pyridyloxy)-phenoxy)-propionsäurebutylester,
2-(4-(6-Chlor-2-chinoxalyloxy)-phenoxy)-propionsäureethylester,
2-(4-(6-Fluor-2-chinoxalyloxy)-phenoxy)-propionsäureethylester,
2-(4-(6-Chlor-2-chinolyloxy)-phenoxy)-propionsäureethylester.

Das Mengenverhältnis Antidot (Verbindung der Formel I) zu Herbizid kann innerhalb weiter Grenzen zwischen 1 : 10 und 10 : 1, insbesondere zwischen 2 : 1 und 1 : 10 schwanken. Die jeweils optimalen Mengen an Herbizid und Antidot sind abhängig vom Typ des verwendeten Herbizids oder vom verwendeten Antidot sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle, Zuckerrüben, Zuckerrohr und Sojabohne.

Die Safener können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein.

Bevorzugt ist jedoch die gleichzeitige Anwendung des Antidots mit dem Herbizid in Form von Tankmischungen oder Fertigformulierungen.

Die Verbindungen der Formel I oder deren Kombination mit einem Herbizid können als Spritzpulver, emulgierbare Konzentrate, Granulate oder Mikrogranulate in den üblichen Zubereitungen angewendet werden.

Unter Spritzpulvern werden in Wasser gleichmäßig dispergierbare Präparate verstanden, die neben dem Wirkstoff oder den Wirkstoffen außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise z. B. durch Mahlen und Vermischen der Komponenten.

Emulgierbare Konzentrate können z. B. durch Auflösen der Wirkstoffe in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil ganz oder auch teilweise entfallen. Als Emulgatoren können beispielsweise verwendet werden: Alkylarylsulfonsaure Calciumsalze wie Cadodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen der Wirkstoffe mit fein verteilten, festen Stoffen, z. B. Talkum, natürlichen Tonen wie Kaolin, Bentonit, Pyrophillit oder Diatomeenerde erhalten.

Granulate können entweder durch Verdüsen der Wirkstoffe auf adsorptionsfähiges, granuliertes Inertmaterial hergestellt werden oder durch Aufbringen von Wirkstoffkonzentraten mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise — gewünschtenfalls in Mischung mit Düngemitteln — granuliert werden.

In Spritzpulvern beträgt die Gesamtwirkstoffkonzentration etwa 1 bis 90 Gew.-%; der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die benötigten Aufwandmengen der Verbindungen der Formel I können je nach Indikation innerhalb weiter Grenzen schwanken und variieren auch in Abhängigkeit von äußeren Bedingungen wie Bodenverhältnissen und Klimabedingungen sowie mit der Art des verwendeten Herbizids. Im allgemeinen liegen sie jedoch zwischen 0,01 und 10 kg Wirkstoff/ha.

Die folgenden Beispiele dienen der weiteren Erläuterung der Erfindung.

Herstellung der Verbindungen der Formel I sowie deren physikalische Daten

1. 1-(4-Chlorphenyl)-2-methylpropanon-O-(4-chlorphenoxyacetyl)-oxim

Zu einer Lösung von 9,9 g (0,05 mol) 1-(4-Chlorphenyl)-2-methyl-propanonoxim und 5,0 g (0,05 mol) Triethylamin in 100 ml wasserfreiem Toluol tropfte man unter Rühren bei 50 °C eine Lösung von 10,3 g

(0,05 mol) 4-Chlorphenoxyacetylchlorid in 20 ml Toluol. Man rührte 8 h bei 40-50 °C, kühlte ab, entfernte das Lösungsmittel am Rotationsverdampfer, nahm den Rückstand in 200 ml Dichlormethan auf und wusch die organische Phase zweimal mit je 100 ml Wasser. Nach Trocknen der organischen Phase mit $Na_2SO_4$, Abdampfen des Dichlormethans und Entfernen von noch anhaftendem Lösungsmittel im Hochvakuum erhielt man 14,7 g (80,5 % d. Th.) 1-(4-Chlorphenyl)-2-methylpropanon-O-(4-chlorphenoxyacetyl)-oxim mit $n_D^{24}$ = 1,583.

2. Cyclohexanon-O-[2-(4-chlorphenoxy)-propionyl]-oxim

Zu einer Lösung von 5,7 g (0,05 mol) Cyclohexanonoxim und 5,0 g (0,05 mol) Triethylamin in 100 ml wasserfreiem Toluol tropfte man unter Rühren bei 50 °C eine Lösung von 11,0 g (0,05 mol) 2-(4-Chlorphenoxy)-propionylchlorid in 20 ml Toluol. Man rührte 8 h bei 40-50 °C, kühlte ab, entfernte das Lösungsmittel am Rotationsversampfer, nahm den Rückstand in 200 ml Dichlormethan auf und wusch die organische Phase zweimal mit je 100 ml Wasser. Nach Trocknen der organischen Phase mit $Na_2SO_4$, Abdampfen des Dichlormethans und Umkristallisation des verbleibenden Feststoffs aus n-Hexan(Diisopropylether (1 : 1) erhielt man 9,6 g (64,4 % d. Th.) Cyclohexanon-O-[2-(4-chlorphenoxy)-propionyl]-oxim vom Schmp. 66-68 °C.

Die in Tabelle I aufgeführten Verbindungen wurden in analoger Weise erhalten.

Tabelle I

Phenoxyalkansäureoximester

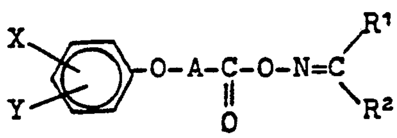

| Vbg. Nr. | X | Y | A | $R^1$ | $R^2$ | Fp [°C], $n_D^{20}$ |
|---|---|---|---|---|---|---|
| 3 | 4-Cl | H | $-CH_2-$ | $-CH_3$ | $-CH_3$ | 1,5394 |
| 4 | 4-Cl | 2-Cl | $-CH_2-$ | $-CH_3$ | $-CH_3$ | 77-79 |
| 5 | 4-Cl | 3-Cl | $-CH_2-$ | $-CH_3$ | $-CH_3$ | 65-67 |
| 6 | 4-Cl | $2-CH_3$ | $-CH_2-$ | $-CH_3$ | $-CH_3$ | 60-61 |
| 7 | 4-Cl | H | $-CH_2-$ | $-CH_3$ | 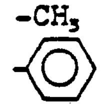 | 82-84 |
| 8 | 4-Cl | 2-Cl | $-CH_2-$ | $-CH_3$ | 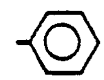 | 80-81 |
| 9 | 4-Cl | 3-Cl | $-CH_2-$ | $-CH_3$ | 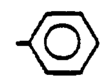 | 89-91 |
| 10 | 4-Cl | $2-CH_3$ | $-CH_2-$ | $-CH_3$ | 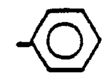 | 87 |
| 11 | 4-Cl | H | $-CH_2-$ | -H | 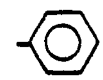 | 96-97 |
| 12 | 4-Cl | 2-Cl | $-CH_2-$ | -H | 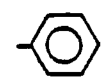 | 102-103 |
| 13 | 3-Cl | H | $-CH_2-$ | -H | 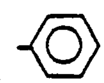 | 82 |
| 14 | 4-Cl | H | $-CH(CH_3)-$ | -H | 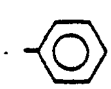 | 91 |
| 15 | 4-Cl | H | $-CH_2-$ | $-CH_3$ | $-CH(CH_3)_2$ | 1,5284 |

Fortsetzung Tabelle I

| Vbg. Nr. | X | Y | A | R¹ | R² | Fp, $n_D^{20}$ |
|---|---|---|---|---|---|---|
| 16 | 4-Cl | 2-Cl | $-CH_2-$ | $-CH_3$ | $-CH(CH_3)_2$ | 1,5420 |
| 17 | 4-Cl | 3-Cl | $-CH_2-$ | $-CH_3$ | $-CH(CH_3)_2$ | 1,5400 |
| 18 | 4-Cl | H | $-CH(CH_3)-$ | $-CH_3$ | $-CH(CH_3)_2$ | 1,5262 |
| 19 | 4-Cl | $2-CH_3$ | $-CH_2-$ | $-CH_3$ | $-CH(CH_3)_2$ | 1,5254 |
| 20 | 4-Cl | H | $-CH_2-$ | $-CH(CH_3)_2$ | 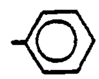 | 1,5670 |
| 21 | 3-Cl | H | $-CH_2-$ | $-CH(CH_3)_2$ | 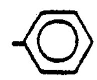 | 1,5650 |
| 22 | 4-Cl | 3-Cl | $-CH_2-$ | $-CH(CH_3)_2$ | 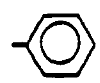 | 1,5745 |
| 23 | 4-Cl | $2-CH_3$ | $-CH_2-$ | $-CH(CH_3)_2$ | 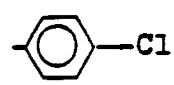 | 1,5685 |
| 24 | 4-Cl | 3-Cl | $-CH_2-$ | $-CH(CH_3)_2$ | 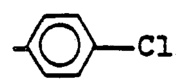 | 1,5765 |
| 25 | 3-Cl | H | $-CH_2-$ | $-CH(CH_3)_2$ | 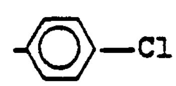 | 1,5702 |
| 26 | 4-Cl | H | $-CH_2-$ | 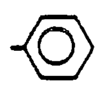 | 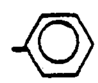 | 102-103 |
| 27 | 3-Cl | H | $-CH_2-$ | 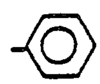 | 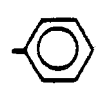 | 93 |
| 28 | 4-Cl | 2-Cl | $-CH_2-$ | 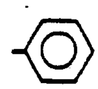 | 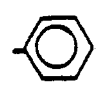 | 70-71 |
| 29 | $4-CH_3$ | H | $-CH_2-$ | 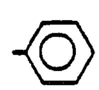 | 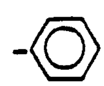 | 98 |
| 30 | 4-Cl | 3-Cl | $-CH_2-$ | 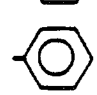 | 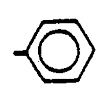 | 101-103 |
| 31 | 4-Cl | H | $-CH(CH_3)-$ | 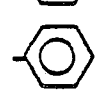 | 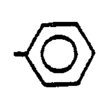 | 83-84 |
| 32 | 4-Cl | H | $-CH_2-$ | 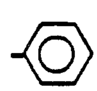 | 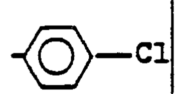 | 94-96 |
| 33 | 4-Cl | 3-Cl | $-CH_2-$ | 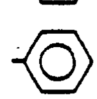 | 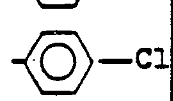 | 78-79 |
| 34 | 4-Cl | H | $-CH(CH_3)-$ | 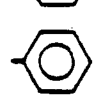 | 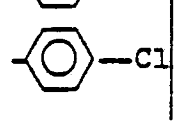 | 96-98 |

Fortsetzung Tabelle I

| Vbg. Nr. | X | Y | A | $R^1$ | $R^2$ | Fp, $n_D^T$ |
|---|---|---|---|---|---|---|
| 35 | 4-Cl | H | $-CH(CH_3)-$ | $-CH_3$ | 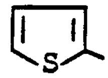 | 86-88 |
| 36 | 4-Cl | H | $-CH_2-$ | $-CH_3$ | 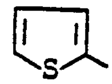 | 80 |
| 37 | 3-Cl | H | $-CH_2-$ | $-CH_3$ | 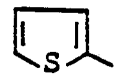 | 76 |
| 38 | 4-Cl | 3-Cl | $-CH_2-$ | $-CH_3$ | 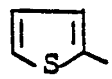 | 104-105 |
| 39 | 4-Cl | H | $-CH_2-$ | $-(CH_2)_5-$ | | 1,5535 |
| 40 | 3-Cl | H | $-CH_2-$ | $-(CH_2)_5-$ | | 69-71 |
| 41 | 4-Cl | 2-Cl | $-CH_2-$ | $-(CH_2)_5-$ | | 1,5642 |
| 42 | 4-Cl | 3-Cl | $-CH_2-$ | $-(CH_2)_5-$ | | 72-74 |
| 43 | 4-$CH_3$ | H | $-CH_2-$ | $-(CH_2)_5-$ | | 51 |
| 44 | 4-Cl | H | $-CH_2-$ | $-(CH_2)_6-$ | | 1,5515 |
| 45 | 4-Cl | 2-Cl | $-CH_2-$ | $-(CH_2)_6-$ | | 1,5590 |
| 46 | 4-Cl | 3-Cl | $-CH_2-$ | $-(CH_2)_6-$ | | 1,5605 |
| 47 | 4-Cl | H | $-CH(CH_3)-$ | $-(CH_2)_6-$ | | 57-58 |
| 48 | 3-Cl | H | $-CH_2-$ | $-(CH_2)_6-$ | | 47 |
| 49 | 4-$CH_3$ | H | $-CH_2-$ | $-(CH_2)_6-$ | | 1,5381 |

Fortsetzung Tabelle I

| Vbg. Nr. | X | Y | A | $R^1$ | $R^2$ | Fp, $n_D^T$ |
|---|---|---|---|---|---|---|
| 50 | 4-Cl | H | $-CH_2-$ | $-(CH_2)_7-$ | | 1,5484 |
| 51 | 4-Cl | 3-Cl | $-CH_2-$ | $-(CH_2)_7-$ | | 73-74 |
| 52 | 4-Cl | 2-$CH_3$ | $-CH_2-$ | $-(CH_2)_7-$ | | 1,5475 |
| 53 | 3-Cl | H | $-CH_2-$ | $-(CH_2)_7-$ | | 40-41 |
| 54 | 4-Cl | H | $-CH-$<br>$CH_3$ | $-CH_3$ | $-CO_2C_2H_5$ | 1,4805 |
| 55 | 4-Cl | H | $-CH_2-$ | $-CH_3$ | $-CO_2C_2H_5$ | 1,4744 |
| 56 | 4-Cl | 3-Cl | $-CH_2-$ | $-CH_3$ | $-CO_2C_2H_5$ | 54 |
| 57 | 4-Cl | H | $-CH-$<br>$CH_3$ | $-CH(CH_3)_2$ | 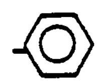 | 78-80 |
| 58 | 4-Cl | H | $-CH_2-$ | H | 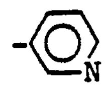 | Oel |

A. Formulierungsbeispiele

1. Ein in Wasser leicht emulgierbares Konzentrat eines Antidots wird erhalten aus

25 Gew.-% Verbindung 3 (der Formel I)
50 Gew.-% Xylol
10 Gew.-% Cyclohexanon
8 Gew.-% dodecylbenzolsulfonsaurem Calcium
4 Gew.-% ethoxyliertem Rizinusöl (40 EO)
3 Gew.-% ethoxyliertem Nonylphenol (10 EO)

Der Wirkstoff wird unter Rühren und leichtem Erwärmen in den angegebenen Lösungsmittelmenge gelöst und anschließend mit den Emulgatoren versetzt. Bei leicht erhöhten Temperaturen wird nachgerührt, bis die Lösung klar und frei von Schlieren ist.

2. Ein in Wasser leicht emulgierbares Konzentrat aus einem Phenoxycarbonsäureester und einem Antidot (10 : 1) wird erhalten aus

12,00 Gew.-% 2-[4-(6-Chlorbenzoxazol-2-yl-oxy)-phenoxy]-propionsäureethylester
1,20 Gew.-% Verbindung 3 (der Formel I)
69,00 Gew.-% Xylol
7,80 Gew.-% dodecylbenzolsulfonsaurem Calcium
6,00 Gew.-% ethoxyliertem Nonylphenol (10 EO)
4,00 Gew.-% ethoxyliertem Rizinusöl (40 EO)

Die Zubereitung erfolgt wie unter Beispiel 1) angegeben.

3. Ein in wasser leicht emulgierbares Konzentrat aus einem Phenoxycarbonsäureester und einem Antidot (1 : 10) wird erhalten aus

4,0 Gew.-% 2-[4-(6-Chlorbenzoxazol-2-yl-oxy)-phenoxy]-propionsäureethylester
40,0 Gew.-% Verbindung 3 der Formel I
30,0 Gew.-% Xylol
20,0 Gew.-% Cyclohexanon
4,0 Gew.-% dodecylbenzolsulfonsaurem Calcium
2,0 Gew.-% ethoxyliertem Rizinusöl (40 EO)

Die Zubereitung erfolgt wie unter Beispiel 1) angegeben.

## B. Biologische Beispiele

### Beispiel 1

Weizen wurde im Gewächshaus in Töpfen von 9 cm Ø bis zum 3-4-Blattstadium herangezogen und dann mit dem Herbizid und den Antidot-Verbindungen behandelt. Das Herbizid wurde dabei zusammen mit dem Antidot in Form wäßriger Suspensionen bzw. Emulsionen als Tankmischung mit einer Wasseraufwandmenge von umgerechnet 800 l/ha ausgebracht. 3 Wochen nach der Behandlung wurden die Pflanzen auf eventuelle Schädigungen durch die ausgebrachten Herbizide bonitiert, wobei insbesondere das Ausmaß der anhaltenden Wachstumshemmung berücksichtigt wurde.

Die Ergebnisse aus Tabelle 1 veranschaulichen, daß die erfindungsgemäßen Mittel starke Herbizidschäden sehr effektiv reduzieren können und sich also zur selektiven Unkrautbekämpfung in Getreide eignen.

Tabelle 1

| Kombination Herbizid/Antidot | Dosis kg a.i./ha | herbizide Wirkung in % TA |
|---|---|---|
| $H_1$ | 2 | 80 |
| $H_1$ + Vbg.Nr. 7 | 2 + 2,5 | 38 |
| $H_1$ + Vbg.Nr. 3 | 2 + 2,5 | 40 |
| $H_1$ + Vbg.Nr. 4 | 2 + 2,5 | 60 |
| $H_1$ + Vbg.Nr. 5 | 2 + 2,5 | 45 |
| $H_1$ + Vbg.Nr. 11 | 2 + 2,5 | 65 |
| $H_1$ + Vbg.Nr. 16 | 2 + 2,5 | 50 |
| $H_1$ + Vbg.Nr. 15 | 2 + 2,5 | 25 |
| $H_1$ + Vng.Nr. 39 | 2 + 2,5 | 20 |
| $H_1$ + Vbg.Nr. 42 | 2 + 2,5 | 60 |
| $H_1$ + Vbg.Nr. 41 | 2 + 2,5 | 30 |
| $H_1$ + Vbg.Nr. 26 | 2 + 2,5 | 35 |
| $H_1$ + Vbg.Nr. 17 | 2 + 2,5 | 20 |
| $H_1$ + Vbg.Nr. 48 | 2 + 2,5 | 60 |
| $H_1$ + Vbg.Nr. 20 | 2 + 2,5 | 40 |
| $H_1$ + Vbg.Nr. 22 | 2 + 2,5 | 50 |
| $H_1$ + Vbg.Nr. 1 | 2 + 2,5 | 50 |
| $H_1$ + Vbg.Nr. 24 | 2 + 2,5 | 50 |
| $H_1$ + Vbg.Nr. 25 | 2 + 2,5 | 60 |
| $H_1$ + Vbg.Nr. 23 | 2 + 2,5 | 60 |

Fortsetzung Tabelle 1

| Kombination Herbizid/Antidot | Dosis kg a.i./ha | herbizide Wirkung in % TA |
|---|---|---|
| $H_1$ + Vbg.Nr. 14 | 2 + 2,5 | 60 |
| $H_1$ + Vbg.Nr. 2 | 2 + 2,5 | 60 |
| $H_1$ + Vbg.Nr. 53 | 2 + 2,5 | 50 |
| $H_1$ + Vbg.Nr. 38 | 2 + 2,5 | 50 |
| $H_1$ + Vbg.Nr. 47 | 2 + 2,5 | 50 |
| $H_1$ + Vbg.Nr. 30 | 2 + 2,5 | 65 |
| $H_1$ + Vbg.Nr. 51 | 2 + 2,5 | 45 |
| $H_1$ + Vbg.Nr. 36 | 2 + 2,5 | 40 |
| $H_1$ + Vbg.Nr. 50 | 2 + 2,5 | 30 |
| $H_1$ + Vbg.Nr. 33 | 2 + 2,5 | 55 |
| $H_1$ + Vbg.Nr. 13 | 2 + 2,5 | 65 |
| $H_1$ + Vbg.Nr. 56 | 2 + 2,5 | 30 |

Abkürzungen :
$H_1$ = Fenoxaprop-ethyl = (2-[4-(6-Chlorbenzoxazol-2-yl-oxy)-phenoxy]-propionsäure-ethylester)
TA = Triticum aestivum
a.i. = Aktivsubstanz

## Patentansprüche

1. Mittel zum Schutz von Kulturpflanzen gegen phytotoxische Nebenwirkungen von Herbiziden, dadurch gekennzeichnet, daß sie als Safener eine Verbindung der Formel I

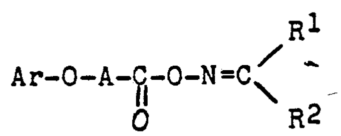

(I)

worin

Ar einen Phenyl-, Naphthyl- oder Chinolylrest, der jeweils bis zu dreifach durch Halogen und/oder ein- oder zweifach durch $CF_3$, ($C_1$-$C_4$)-Alkyl, ($C_1$-$C_4$)-Alkoxy, CN, $NO_2$, ($C_1$-$C_8$-Alkyl)-carbonyl oder Benzoyl substituiert sein kann,

A eine lineare oder verzweigte Kohlenwasserstoffkette mit bis zu vier C-Atomen,

$R^1$ H, ($C_1$-$C_6$)-Alkyl, CN, ($C_1$-$C_4$-Alkoxy)-carbonyl oder Phenyl, das durch ein bis drei Halogenatome, ($C_1$-$C_4$)-Alkyl, ($C_1$-$C_4$)-Alkoxy, $CF_3$, CN, $NO_2$, ($C_1$-$C_4$-Alkyl)-carbonyl, Benzoyl oder durch einen gegebenenfalls durch Halogen, ($C_1$-$C_4$)-Alkoxy, ($C_1$-$C_4$)-Alkyl, Nitro, $CF_3$ oder CN ein- oder mehrfach substituierten Phenyl- oder Phenoxyrest substituiert sein kann ;

$R^2$ die Bedeutung von $R^1$ außer Wasserstoff besitzt oder einen Heterocyclus mit 5 oder 6 Ringgliedern, der ein- bis drei N-, O- oder S-Atome enthalten kann, und durch Halogen oder ($C_1$-$C_4$)-Alkyl ein- bis dreifach substituiert sein kann, oder

$R^1$ und $R^2$ gemeinsam eine Alkylenkette mit 4-7 C-Atomen bedeuten, in Kombination mit Phenoxyphenoxy- oder Heteroaryloxyphenoxy-carbonsäureester-Herbizid enthalten.

2. Mittel gemäß Anspruch 1, dadurch gekennzeichnet, daß in Formel I

Ar Phenyl, das durch ein oder zwei Halogenatome substituiert ist, und

A $-CH_2-$ oder 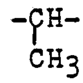

bedeuten.

3. Mittel gemäß Ansprüchen 1 und 2, dadurch gekennzeichnet, daß das Verhältnis Safener zu Herbizid 1 : 10 bis 10 : 1 beträgt.

4. Mittel gemäß Ansprüchen 1 bis 3, dadurch gekennzeichnet, daß das Verhältnis Antidot zu Herbizid 2 : 1 bis 1 : 10 beträgt.

5. Mittel gemäß Ansprüchen 1 bis 4, dadurch gekennzeichnet, daß sie als Herbizid 2-[4-(6-Chlorbenzoxazol-2-yloxy)-phenoxy]propionsäureethylester enthalten.

6. Mittel gemäß Ansprüchen 1 bis 5, dadurch gekennzeichnet, daß sie Verbindungen der Formel I, worin X = 4-Cl, Y = H oder 3-Cl, A = $CH_2$, $R_1$ = H oder $CH_3$ und $R^2$ = Phenyl oder $R^1$, $R^2$, zusammen = $-(CH_2)_5-$ bedeuten, enthalten.

7. Verfahren zur Minderung der Phytotoxizität von Phenoxyphenoxy- oder Heteroaryloxyphenoxy-carbonsäureester-Herbiziden gegenüber Kulturplanzen, dadurch gekennzeichnet, daß man die Pflanzen, Planzensamen oder Anbauflächen mit einer wirksamen Menge eines Mittels von Ansprüchen 1 bis 6 behandelt.

8. Verwendung von Verbindungen der Formel I von Ansprüchen 1, 2 oder 6 zur Minderung der Phytotoxizität von Phenoxyphenoxy- oder Heteroaryloxyphenoxy-Carbonsäureester-Herbiziden gegenüber Kulturpflanzen.

9. Verwendung gemäß Anspruch 8, dadurch gekennzeichnet, daß das Herbizid 2-[4-(6-Chlorbenzoxazol-2-yloxy)-phenoxy]propionsäureethylester ist.

## Claims

1. An agent for protecting crop plants from the phytotoxic side effects of herbicides, this agent containing as safener a compound of the formula I

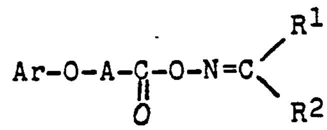

in which

Ar denotes a phenyl, naphthyl or quinolyl radical, each of which can be mono-, di- or tri-substituted by halogen and/or mono- or di-substituted by $CF_3$, $(C_1$-$C_4)$-alkyl, $(C_1$-$C_4)$-alkoxy, CN, $NO_2$, $(C_1$-$C_8$-alkyl)-carbonyl or benzoyl,

A denotes a linear or branched hydrocarbon chain with up to four carbon atoms,

$R^1$ denotes H, $(C_1$-$C_6)$-alkyl, CN or $(C_1$-$C_4$-alkoxy)-carbonyl, or phenyl, which can be substituted by one to three halogen atoms, $(C_1$-$C_4)$-alkyl, $(C_1$-$C_4)$-alkoxy, $CF_3$, CN, $NO_2$, $(C_1$-$C_4$-alkyl)-carbonyl or benzoyl or by a phenyl or phenoxy radical each of which is optionally mono- or poly-substituted by halogen, $(C_1$-$C_4)$-alkoxy, $(C_1$-$C_4)$-alkyl, nitro, $CF_3$ or CN, and

$R^2$ has the meaning of $R^1$, with the exception of hydrogen, or denotes a heterocyclic radical which has 5 or 6 ring members, can contain one to three N, O or S atoms and can be mono-, di- or tri-substituted by halogen or $(C_1$-$C_2)$-alkyl, or

$R^1$ and $R^2$ together denote an alkylene chain with 4-7 carbon atoms, in combination with a phenoxyphenoxy- or heteroaryloxyphenoxy-carboxylic acid ester herbicide.

2. An agent as claimed in claim 1, wherein, in formula I,

Ar denotes phenyl, which is substituted by one or two halogen atoms, and

A denotes $-CH_2-$ or 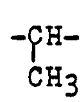

3. An agent as claimed in either of claims 1 and 2, wherein the ratio of safener to herbicide is 1 : 10 to 10 : 1.

4. An agent as claimed in any one of claims 1 to 3, wherein the ratio of antidote to herbicide is 2 : 1 to 1 : 10.

5. An agent as claimed in any one of claims 1 to 4, which contains ethyl 2-[4-(6-chlorobenzoxazol-2-yl-oxy)-phenoxy]-propionate as the herbicide.

6. An agent as claimed in any one of claims 1 to 5 which contains compounds of the formula I in which X denotes 4-Cl, Y denotes H or 3-Cl, A denotes $CH_2$, $R^1$ denotes H or $CH_3$ and $R^2$ denotes phenyl or $R^1$ and $R^2$ together denote $-(CH_2)_5-$.

7. A method of reducing the phytotoxicity of a phenoxyphenoxy- or heteroaryloxyphenoxy-carboxylic acid ester herbicide towards crop plants, which comprises treating the plants, plant seeds or areas for cultivation with an effective amount of an agent of any one of claims 1 to 6.

8. The use of a compound of the formula I of any one of claims 1, 2 or 6 for reducing the phytotoxicity of a phenoxyphenoxy- or heteroaryloxyphenoxy carboxylic acid ester herbicide towards crop plants.

9. The use as claimed in claim 8, wherein the herbicide is ethyl 2-[4-(6-chlorobenzoxazol-2-yl-oxy)-phenoxy]-propionate.

## Revendications

1. Produits destinés à protéger des plantes de culture contre les effets secondaires phytotoxiques d'herbicides, produits caractérisés en ce qu'ils contiennent comme détoxicant un composé de formule I ci-dessous :

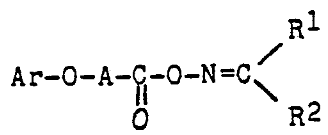

(I)

dans laquelle :

Ar désigne un radical phényle, naphtyle ou quinolyle pouvant avoir chacun comme substituants jusqu'à 3 atomes d'halogènes et/ou un ou deux groupes $CF_3$, $(C_1$-$C_4)$-alkyles, $(C_1$-$C_4)$-alcoxy, CN, $NO_2$, $(C_1$-$C_8$-alkyl)-carbonyles ou benzoyles,

A désigne une chaîne hydrocarbonée linéaire ou ramifiée avec jusqu'à 4 atomes de carbone,

$R^1$ l'hydrogène, un alkyle en $C_1$-$C_6$, le groupe CN, un $(C_1$-$C_4$-alcoxy)-carbonyle ou un phényle, pouvant avoir comme substituants de 1 à 3 atomes d'halogènes ou groupes alkyles en $C_1$-$C_4$, alcoxy en $C_1$-$C_4$, $CF_3$, CN, $NO_2$ $(C_1$-$C_4$-alkyl)-carbonyles ou benzoyles ou encore un radical phényle ou phénoxy avec éventuellement comme substituants un ou plusieurs atomes d'halogènes ou groupes alcoxy en $C_1$-$C_4$, alkyles en $C_1$-$C_4$, nitro, $CF_3$ ou CN, et

$R^2$ a la même signification que $R^1$ à l'exception de l'hydrogène ou représente un hétérocycle à 5 ou 6 chaînons comprenant de 1 à 3 atomes d'azote, d'oxygène ou de soufre et pouvant porter de 1 à 3 atomes d'halogènes ou alkyles en $C_1$-$C_4$, ou bien

$R^1$ et $R^2$ forment ensemble une chaîne alkylène en $C_4$ à $C_7$, détoxicant qui est associé à un herbicide d'ester d'acide phénoxyphénoxy-carboxylique ou hétéroaryloxy-phénoxycarboxylique.

2. Produits selon la revendication 1, caractérisés en ce que la formule I

Ar est le radical phényle avec un ou deux atomes d'halogène et

A représente le groupe $-CH_2-$ ou $-CH(CH_3)-$

3. Produits selon les revendications 1 et 2, caractérisés en ce que le rapport du détoxicant à l'herbicide est compris entre 1 : 10 et 10 : 1.

4. Produits selon les revendications 1 et 3, caractérisés en ce que le rapport de l'antidote à l'herbicide est compris entre 2 : 1 et 1 : 10.

5. Produits selon les revendications 1 à 4, caractérisés en ce qu'ils contiennent comme herbicide du 2-[4-(6-chlorobenzoxazol-2-yloxy)-phénoxy]-propionate d'éthyle.

6. Produits selon les revendications 1 à 5, caractérisés en ce qu'ils contiennent des composés de formule I dans lesquels X est un atome de chlore à la position 4, Y l'hydrogène ou un atome de chlore à la position 3, A le groupe $CH_2$, $R^1$ l'hydrogène ou le groupe $CH_3$ et $R^2$ le groupe phényle, ou bien $R^1$ et $R^2$ forment ensemble un groupe $—(CH_2)_5—$.

7. Procédé pour abaisser la phytotoxicité d'herbicides à base d'esters d'acides phénoxyphénoxy-carboxyliques ou hétéroaryloxyphénoxy-carboxyliques à l'égard de plantes cultivées, procédé caractérisé en ce que l'on traite les plantes, leurs semences ou les surfaces de culture avec une quantité appropriée d'un produit selon les revendications 1 à 6.

8. Emploi de composés de formule I selon les revendications 1, 2 ou 6 pour abaisser la phytotoxicité à l'égard de plantes cultivées d'herbicides d'esters d'acides phénoxyphénoxy-carboxyliques ou hétéroaryloxyphénoxy-carboxyliques.

9. Emploi selon la revendication 8, caractérisé en ce que l'herbicide est le 2-[4-(6-chlorobenzoxazol-2-yloxy)-phénoxy]-proprionate d'éthyle.